# FASCICULE DE BREVET EUROPEEN

(11) **EP 0 815 901 B1**
(45) Date de publication et mention de la délivrance du brevet: **02.10.2002**
(21) Numéro de dépôt: 97401512.5
(22) Date de dépôt: 27.06.1997
(51) Int. Cl.: A61N 7/02, A61B 8/12

(54) **Sonde de thérapie**
Therapiesonde
Therapy probe

(30) Priorité: 28.06.1996 FR 9608096
(43) Date de publication de la demande: 07.01.1998
(73) Titulaire: TECHNOMED MEDICAL SYSTEMS, 69120 Vaulx-en-Velin (FR); INSTITUT NATIONAL DE LA SANTE ET DE LA RECHERCHE MEDICALE (INSERM), 75654 Paris Cédex 13 (FR)
(72) Inventeur: Chapelon, Jean-Yves, 69100 Villeurbanne (FR); Blanc, Emmanuel, 69230 ST Genis Laval (FR); Lacoste, François, 92500 Rueil Malmaison (FR)
(74) Mandataire: Desrousseaux, Grégoire Marie

(56) Documents cités:
- WO-A-93/16641
- WO-A-95/02994
- DE-C- 4 302 537
- US-A- 4 858 613

## Description

la présente invention a pour objet une sonde de thérapie, comprenant un transducteur de thérapie, monté mobile sur un corps de sonde.

De telles sondes s'appliquent notamment au traitement de la prostate par ultrasons focalisés, par voie rectale. Elles s'appliquent aussi au traitement de l'oesophage, de l'estomac, ou du foie, et plus généralement aux traitements percutanés ou par laparoscopie.

Ces sondes comprennent avantageusement aussi un transducteur d'imagerie, qui permet une visualisation de la zone traitée pendant le traitement.

WO-A-8907909 décrit une sonde de thérapie et d'imagerie pour une utilisation endocavitaire, comprenant un transducteur de thérapie mobile en rotation et un transducteur d'imagerie mobile en translation; les mouvements de translation et de rotation sont indépendants.

FR-A-2 673 542 (WO-A-9215523) décrit une sonde de thérapie et d'imagerie, comprenant un transducteur de thérapie en forme de cuillère, auquel est associé un transducteur d'imagerie. Les deux transducteurs ne se déplacent pas l'un par rapport à l'autre.

FR-A-2 708 207 décrit une sonde de thérapie et d'imagerie, comprenant un transducteur de thérapie en forme de cuillère. Le transducteur de thérapie est mobile en rotation et le transducteur d'imagerie est mobile en translation. Les deux transducteurs sont disposés dans une enveloppe souple commune

FR-A-2 715 822 décrit une sonde de thérapie et d'imagerie dans laquelle le transducteur de thérapie et le transducteur d'imagerie sont mobiles et entraînés par des moyens d'entraînement communs; les deux transducteurs sont mobiles à l'intérieur d'une enveloppe rigide présentant une fenêtre. WO-A-95 02294 revendique la priorité de FR-A-2 708 207 et de FR-A-2 715 822.

WO-A-93 16641 décrit une sonde endocavitaire, avec un transducteur mobile en translation et en rotation à l'intérieur d'un corps de sonde rigide. Il est prévu une fenêtre dans le corps de sonde. Ce dispositif est du même genre que celui de FR-A-2 715 822.

N. Bom et al, Early and recent intraluminal ultrasound devices, International Journal of Cardiac Imaging, 4:79-88 1989 décrit divers types de sondes d'imagerie ou de traitement.

Le but de toutes les sondes de thérapie est d'assurer une thérapie aussi efficace et précise que possible; si un transducteur d'imagerie est prévu, un autre but est de permettre une visualisation de la zone traitée, de préférence aussi au cours du traitement. Enfin, dans le cas de sondes endocavitaires, le volume total de la sonde ou sa section est aussi un paramètre important.

L'invention résulte de la découverte de problèmes nouveaux des sondes connues et propose des solutions permettant de résoudre ces problèmes. Certains de ces problèmes s'appliquent à toutes les sondes, qu'elles présentent ou non un transducteur d'imagerie. D'autres problèmes ne se posent que si les sondes présentent un transducteur de thérapie et un transducteur d'imagerie.

Un problème résolu par l'invention réside dans la détection des mouvements du patient. Dans les documents de l'art antérieur cités sont décrits de nombreux systèmes de maintien et de guidage de la sonde. Aucun de ces documents ne décrit le problème nouveau des mouvements du patient lors du traitement. Or ces mouvements peuvent conduire à un déplacement de la sonde par rapport au patient ou par rapport à l'organe à traiter ou à visualiser. L'invention propose une solution à ce nouveau problème.

Un autre problème résolu par l'invention réside dans le guidage et le maintien de la sonde par rapport au patient. Dans le document WO-A-8907909. pour le traitement de la prostate, il est proposé de déplacer la sonde introduite dans le rectum en rotation autour de son axe général. Dans FR-A-2 673 542, pour le traitement de la prostate, il est prévu une sonde endorectale de traitement et d'imagerie et une sonde endo-urétrale d'imagerie: au cours de la mise en place des deux sondes, il est proposé de déplacer les sondes dans le plan de symétrie du patient, pour les rapprocher ou les éloigner l'une de l'autre; la position relative des sonde est ensuite gelée pour le traitement. En cours de traitement, les deux sondes sont déplacées indépendamment l'une de l'autre en rotation autour de leur axe longitudinal respectif, et sont déplacées ensemble en translation le long de leurs axes longitudinaux parallèles, i.e. essentiellement parallèlement au rectum. FR-A-2 708 207 propose une sonde montée en translation suivant trois directions, et en rotation autour de son axe longitudinal. FR-A-2 715 822 décrit une sonde montée en rotation autour de son axe longitudinal dans un collier fixé à une extrémité d'une tige. L'autre extrémité de la tige est montée dans un joint rotule, pour permettre une rotation dans un plan vertical. Le joint rotule est monté en translation suivant deux axes dans le même plan vertical.

L'invention résoud de nouveaux problèmes liés à ces dispositifs: d'une part, le mouvement dans la direction de propagation des ultrasons n'est pas prévu pendant le traitement; dans le cas de la prostate, on se contente de déplacer la sonde dans la direction du rectum au cours du traitement. Ceci pose des problèmes pour un traitement précis et complet de l'organe.

D'autre part, la rotation de la sonde autour de son axe longitudinal pose problème, dans la mesure où elle peut conduire à des déplacement de l'organe à traiter. tout particulièrement si la sonde n'a pas une section cylindrique; ceci est par exemple le cas de FR-A-2 708 207, ou encore de FR-A-2 715 822, lorsque l'enveloppe souple est gonflée. L'invention propose une solution à ces nouveaux problèmes.

Un autre problème résolu par l'invention réside dans l'introduction de la sonde. Dans le cas par exemple du traitement de la prostate par voie rectale. il est nécessaire d'introduire la sonde dans le rectum du patient, qui n'est pas rectiligne. Ce problème se pose aussi pour les traitements endocavitaires. percutanés ou par laparoscopie d'autres organes, comme par exemple l'oesophage. l'estomac ou le foie. dans lequel la sonde doit aussi être introduite dans une ouverture qui n'est pas nécessairement rectiligne. Les sondes de l'art antérieur n'apportent aucune solution à ce problème, qui n'est pas mentionné dans les documents cités.

Encore un problème résolu par l'invention réside dans les mouvements de la sonde par rapport à l'organe à traiter. Comme mentionné plus haut, la rotation de la sonde autour de son axe longitudinal pose problème, dans la mesure où elle peut conduire à des déplacements de l'organe à traiter, tout particulièrement si la sonde n'a pas une section cylindrique. Ce problème particulier ne se pose pas dans la sonde selon FR-A-2 715 822, dans laquelle le transducteur de thérapie et le transducteur d'imagerie sont disposés dans un corps de sonde rigide et cylindrique; mais cette solution conduit à augmenter le diamètre de la sonde, ou à diminuer la taille du transducteur de thérapie. L'invention apporte une solution à ce nouveau problème. sans pour autant impliquer de diminution de la taille du transducteur de thérapie ni d'augmentation de la section de la sonde.

L'invention apporte une solution au nouveau problème des mouvements relatifs de la sonde et de l'organe à traiter, lorsque le transducteur de thérapie se déplace par rapport à la sonde.

Ces problèmes nouveaux se posent pour tous les type de sondes, de thérapie, ou de thérapie et d'imagerie, voire même seulement d'imagerie.

Enfin, l'invention apporte une solution au nouveau problème des mouvements relatifs du transducteur de thérapie et du transducteur d'imagerie, dans un espace restreint, et sans impliquer de déplacement des organes à traiter ou de réduction de la taille du transducteur de thérapie.

L'ensemble de ces nouveaux problèmes est résolu par l'invention, qui propose une sonde de thérapie, comprenant un transducteur de thérapie monté mobile par rapport à un corps de sonde entre une position de thérapie et une position escamotée, dans une enveloppe au moins partiellement déformable lorsque le transducteur se déplace, et un anneau de garde enveloppé dans l'enveloppe limitant en amplitude les déformations de l'enveloppe produites au voisinage de la position du transducteur lorsque le transducteur se déplace par rapport au corps de sonde de la position de thérapie à la position escamotée.

Dans un mode de réalisation, le transducteur de thérapie est mobile entre une position de thérapie et une position escamotée, et l'anneau de garde constitue une butée sur laquelle le transducteur de thérapie vient en appui en position de thérapie.

On peut prévoir des moyens de maintien du transducteur en position de thérapie contre l'anneau de garde.

Dans un mode de réalisation, l'anneau de garde a la forme d'un anneau entourant le transducteur de thérapie en position de thérapie.

Dans un autre mode de réalisation, l'anneau de garde a la forme d'un disque recouvrant sensiblement le transducteur de thérapie en position de thérapie.

Il est possible de monter l'anneau de garde sur le corps de sonde, ou encore sur un support du corps de sonde.

Dans un autre mode de réalisation, indépendamment brevetable, la sonde comprend un transducteur de thérapie, et un transducteur d'imagerie, montés mobile sur un corps de sonde; le transducteur de thérapie est mobile entre une position de thérapie et une position escamotée, et le transducteur d'imagerie est mobile entre une position rétractée et une position d'imagerie dans laquelle le transducteur de thérapie est en position escamotée; le transducteur de thérapie passe de sa position de thérapie à sa position escamotée sous l'action transducteur d'imagerie lorsque celui-ci passe de sa position rétractée à sa position d'imagerie, tandis que le transducteur de thérapie passe de sa position escamotée à sa position de thérapie sous l'action de moyens de rappel lorsque le transducteur d'imagerie passe de sa position d'imagerie à sa position rétractée.

Dans ce cas, on peut prévoir que le transducteur d'imagerie est mobile en translation parallèlement à l'axe longitudinal de la sonde.

Le transducteur de thérapie peut être mobile en translation le long d'un axe sensiblement perpendiculaire à l'axe longitudinal de la sonde.

Les moyens de rappel peuvent comprendre des lames formant ressort.

Il est aussi possible que le transducteur de thérapie soit mobile en rotation autour d'un axe sensiblement parallèle à l'axe longitudinal de la sonde.

Dans un mode de réalisation, les moyens de rappel comprennent un ressort de torsion.

Dans ce mode de réalisation, on pourrait aussi prévoir un anneau de garde.

Dans encore un autre mode de réalisation indépendamment brevetable, la sonde comprend un transducteur de thérapie, et un transducteur d'imagerie, le transducteur d'imagerie est mobile entre une position rétractée et une position d'imagerie montés mobile sur un corps de sonde, et le transducteur de thérapie présente une encoche pour recevoir le transducteur d'imagerie en position rétractée.

Dans ce cas, l'encoche peut être ménagée, de préférence par fraisage, sur le côté du transducteur de thérapie le plus proche du corps de sonde.

La sonde peut être montée sur une rotule.

La sonde de thérapie peut aussi, de façon indépendamment brevetable. être montée sur des moyens d'entraînement en translation dans une direction sensiblement perpendiculaire à la direction principale de propagation des ondes de thérapie et sensiblement perpendiculaire à l'axe longitudinal de la sonde.

Il est aussi avantageux de prévoir qu'une sonde de thérapie soit montée sur des moyens d'entraînement en translation dans trois directions perpendiculaires deux à deux, et que lesdits moyens d'entraînement soient débrayables pour permettre un entraînement manuel au moins dans un plan.

De façon indépendamment brevetable, on peut monter la sonde sur des moyens de déplacement qui déplacent la sonde avec des pas différents dans les différentes directions.

Dans un mode de réalisation, la sonde comprend des moyens de circulation d'un liquide, de préférence thermostaté, dans l'enveloppe. Ces moyens de circulation peuvent comprendre au moins deux canaux disposés dans le corps de sonde.

L'invention a aussi pour objet un procédé de détection des mouvements d'un patient maintenu sur une table de traitement, consistant à disposer une barrière optique entre le patient et la table.

La barrière optique peut comprendre un réflecteur fixé sur la patient et un émetteur récepteur fixé sur la table.

Ces différents modes de réalisation sont indépendants, mais peuvent à l'évidence être combinés.

D'autres caractéristiques et avantages de l'invention apparaîtront à la description qui suit de modes de réalisation de l'invention, donnés à titre d'exemple uniquement et en référence aux dessins qui montrent:
figure 1 une vue de face d'une sonde selon un premier mode de réalisation de l'invention, en position d'imagerie;
figure 2, une vue de côté de la sonde de la figure 1, en position d'imagerie;
figure 3, une vue de dessus en coupe de la sonde de la figure 1, en position d'imagerie;
figure 4, une vue de côté de la sonde de la figure 1, en position de tir;
figure 5, une vue de face d'un transducteur de thérapie selon un deuxième mode de réalisation de l'invention;
figure 6, une vue de dessous du transducteur de la figure 5;
figure 7, une vue de dessous en coupe partielle du transducteur de thérapie des figures 5 et 6. avec le transducteur d'imagerie correspondant;
figure 8, une vue de côté en coupe partielle d'une sonde selon l'invention, en position de tir;
figure 9, une vue schématique en coupe d'une sonde de l'art antérieur, en position de tir et en position d'imagerie;
figure 10, une vue schématique en coupe d'une sonde selon un troisième mode de réalisation de l'invention. en position de tir et en position d'imagerie;
figure 11. une vue schématique d'un quatrième mode de réalisation de l'invention:
figure 12, une vue schématique d'un cinquième mode de réalisation de l'invention;
figure 13, une vue schématique du cinquième mode de réalisation de l'invention, en coupe comme dans les figures 9 et 10;
figures 14 et 15, deux vues schématiques en coupe de sondes selon l'invention. en position pour un traitement de la prostate par voie rectale;
figure 16, une vue schématique d'un patient en position de traitement par une sonde selon l'invention.

Dans l'ensemble des figures, on a représenté des sondes de thérapie présentant, outre le transducteur de thérapie, un transducteur d'imagerie. L'invention s'applique aussi lorsque la sonde ne présente qu'un transducteur de thérapie, comme expliqué en référence aux figures 9 à 11.

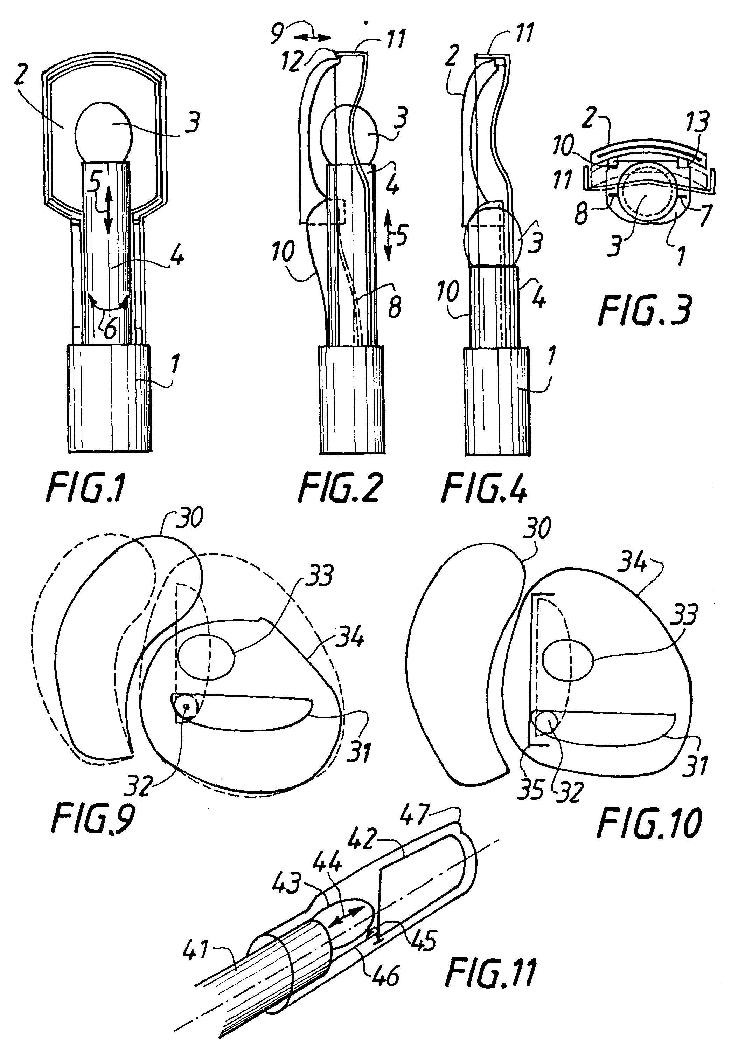

La figure 1 montre une vue de face d'une sonde selon un premier mode de réalisation de l'invention, en position d'imagerie; la figure 1 ne représente que l'extrémité frontale de la sonde; on reconnaît sur la figure 1 l'extrémité d'un corps de sonde 1, un transducteur de thérapie 2, et un transducteur d'imagerie 3.

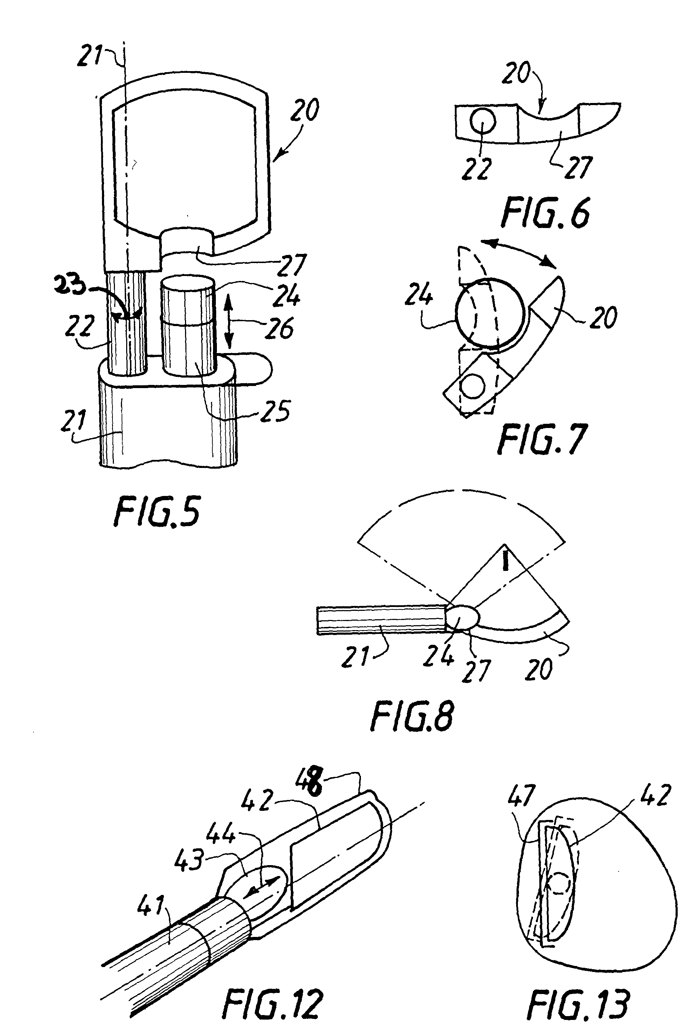

Le transducteur de thérapie 2 est mobile par rapport au corps de sonde 1 entre une position de thérapie ou position de tir, figure 4 et une position escamotée, figure 2, permettant l'imagerie. Le transducteur de thérapie 2 est monté sur le corps de sonde par des lames 7, 8 flexibles dans un plan perpendiculaire au plan de la figure 1; ainsi le transducteur de thérapie est mobile en translation sensiblement perpendiculairement au plan de la figure, i.e. sensiblement perpendiculairement à l'axe longitudinal de la sonde. Le transducteur de thérapie est monté sur le corps de sonde par quatre lames flexibles, situées de part et d'autre du support 4 de la sonde d'imagerie, en avant de celui-ci - lames 7 et 8- et en arrière de celui ci - et lames 10 et 13, sur la figure 2 et la figure 3.

Le transducteur d'imagerie 3 est monté sur un support 4 mobile en translation par rapport au corps de sonde 1, comme indiqué par la flèche 5, entre une position rétractée. figure 4, et une position d'imagerie, figure 1 ou 2. Dans un mode de réalisation, le transducteur d'imagerie est aussi monté mobile en rotation, autour d'un axe parallèle à l'axe longitudinal de la sonde, de sorte à effectuer un balayage dans un plan transversal perpendiculaire à l'axe longitudinal de la sonde, comme indiqué par la flèche 6. On assure ainsi la possibilité d'un balayage complet de la zone à traiter. La rotation du transducteur d'imagerie peut être facilement obtenue par rotation du support 4.

Le transducteur de thérapie est dans le mode de réalisation de la figure 1, un transducteur de thérapie en forme de cuillère, du type de celui de la demande FR-A-2 673 542, auquel on pourra se référer pour plus de détails sur cette question. On peut aussi utiliser pour le transducteur d'imagerie les transducteurs décrits dans les demandes FR-A-2 673 542 ou FR-A-2 708 207.

La figure 2 montre une vue de côté de la sonde de la figure 1, en position d'imagerie. On reconnaît sur la figure 2 les éléments déjà décrits en référence à la figure 1, ainsi que la troisième lame 10 supportant le transducteur de thérapie. La flèche 9 montre la direction du mouvement du transducteur de thérapie entre la position de tir et la position escamotée.

Comme on peut le voir sur la figure 2, dans la position d'imagerie, le transducteur d'imagerie, par l'intermédiaire de son support 4, repousse le transducteur de thérapie, pour maintenir celui ci dans sa position escamotée.

La figure 2 montre encore un anneau de garde 11, agissant aussi comme butée, sur lequel le transducteur de thérapie s'appuie dans la position de traitement. La nature et la fonction de cet anneau de garde apparaîtront plus clairement de la description des figures 9 et 10. Des moyens de maintien, tels des aimants 12 permettent de maintenir le transducteur de thérapie dans la position de tir; les moyens de maintien peuvent aussi, le cas échéant, être utilisés pour détecter la présence du transducteur de thérapie en position de tir.

La figure 3 montre une vue de dessus en coupe de la sonde de la figure 1, en position d'imagerie. Apparaît en pointillés sur la figure 3 la position de tir du transducteur de thérapie, en butée contre l'anneau de garde 11. On voit sur la figure 3 les quatre lames 7, 8, 10, 13 supportant le transducteur de thérapie.

La figure 4 montre une vue de côté de la sonde de la figure 1, en position de tir; dans cette position de tir, le transducteur d'imagerie est en position rétractée, et le transducteur de thérapie est ramené sous l'action des lames flexibles dans la position de tir, dans laquelle il est en butée contre l'anneau de garde.

Le fonctionnement de la sonde des figures 1 à 4 est le suivant. Pour une utilisation endocavitaire, le transducteur d'imagerie est ramené en position rétractée par translation du support 4 vers le corps de sonde. Le transducteur est alors ramené sous l'action des lames flexibles vers l'anneau de garde 11, dans la position de thérapie représentée à la figure 4. La sonde de l'invention présente alors une section minimale permettant une introduction aisée dans une cavité tel que par exemple le rectum d'un patient.

Une fois la sonde positionnée sensiblement face à l'organe à traiter, on fait avancer le transducteur d'imagerie depuis sa position rétractée (figure 4) vers sa position d'imagerie (figure 3). Le déplacement du transducteur d'imagerie 3 provoque le déplacement du transducteur depuis sa position de tir (figure 4) vers sa position escamotée (figure 2). Dans le mode de réalisation de la figure 1. le transducteur d'imagerie glisse le long de la surface intérieure du transducteur et sollicite celui-ci à l'encontre des lames flexibles formant des moyens de rappel.

On peut alors procéder à une visualisation de l'organe à traiter, grâce au transducteur d'imagerie, en tournant au besoin le support 4 du transducteur d'imagerie. Cette visualisation permet un positionnement précis de la sonde.

Pour procéder au traitement, le transducteur d'imagerie est ramené en position rétractée sans déplacer la sonde, et le transducteur de thérapie reprend sous l'action des moyens de rappel sa position de tir (figure 4). On peut alors procéder au traitement, tout en assurant une visualisation, comme expliquée en référence à la figure 8. La présence de l'anneau de garde 11 assure que la paroi de l'organe à traiter reste bien immobile au cours du mouvement des transducteurs dans la sonde, comme expliqué en référence aux figures 10 à 12. On peut ensuite repasser en position d'imagerie, puis de tir, suivant le protocole de traitement souhaité.

On peut employer pour commander ou détecter les mouvements du transducteur d'imagerie tous les moyens connus de l'homme de l'art, tels que moteurs pas à pas, pignons et crémaillère, vis sans fin, vérin etc. De même, pour détecter la position du transducteur de thérapie,, on peut employer les moyens connus de l'homme du métier. Pour plus de détails sur ce sujet, on pourra se référer aux demandes FR-A-2 673 542, FR-A-2 708 207 ou FR-A-2 715 822.

On n'a pas décrit en référence aux figures 1 à 4 ni les moyens de commande ou de pilotage des transducteurs d'imagerie et de thérapie, pour lequel l'homme de l'art peut employer tous les moyens connus. On n'a pas non plus décrit l'enveloppe externe souple enveloppant l'ensemble des transducteurs et l'anneau de garde, qui peut être avantageusement semblable à celle de FR-A-2 673 542 et FR-A-2 708 207.

Par rapport aux sondes des documents cités, la sonde des figures 1 à 4 combine une section faible, facilitant l'introduction dans une cavité et une simplicité dans la commande des mouvements relatifs des transducteurs. De fait, il n'est plus nécessaire de disposer de moyens d'entraînement indépendants pour le transducteur d'imagerie et le transducteur de thérapie: ceci simplifie la structure de la sonde et son volume total. La présence de l'anneau de garde assure un bon maintien de la sonde par rapport à l'organe à traiter. Le déplacement du transducteur de thérapie est plus limité que dans les solutions connues, ce qui permet un traitement dans un volume plus restreint, et sans déplacer l'organe à traiter quand on passe de la position de tir à la position escamotée, ou inversement.

Dans le mode de réalisation des figures 1 à 4, le transducteur de thérapie est monté sur des moyens de rappel constitués de lames flexibles. On peut utiliser d'autres moyens de rappels tels que des biellettes associés à des ressorts. On peut aussi prévoir un rappel du transducteur de sa position escamotée vers la position de tir par les moyens d'entraînement du transducteur d'imagerie après que celui-ci est parvenu en position rétractée.

La figure 5 montre une vue de face d'une sonde selon un deuxième mode de réalisation de l'invention. Le transducteur de thérapie 20 de la figure 5 est monté mobile par rapport à un corps de sonde 21, en rotation par rapport à l'arbre 22. comme symbolisé par la flèche 23. Le transducteur d'imagerie 24 est monté sur un support 25 mobile en translation par rapport un corps de sonde 21, comme symbolisé par la flèche 26.

Dans sa partie la plus proche du corps de sonde 21, et face au transducteur d'imagerie 24, le transducteur de thérapie présente une encoche 27, par exemple ménagée par fraisage, pour faciliter le passage du transducteur d'imagerie.

La figure 6 montre une vue de dessous du transducteur 20 de la figure 5. On reconnaît sur la figure 6 l'arbre 22, et le fraisage 27. Le transducteur 20 présente une forme de cuillère autofocalisante, comme décrit dans FR-A-2 673 542.

Dans un mode de réalisation l'arbre 22 est commandé en rotation par rapport au corps de sonde 21 par des moyens d'entraînement, comme dans la demande FR-A-2 673 542. Toutefois, dans un mode de réalisation préféré, le transducteur de thérapie n'est pas mobile indépendamment du transducteur d'imagerie, et est simplement sollicité par des moyens de rappel. Par exemple, l'arbre 22 est monté en rotation dans le corps 21 sur une plage angulaire limitée et est sollicité par un ressort de torsion vers l'une des positions limite de la plage angulaire, correspondant à la position de tir.

La figure 7 montre une vue de dessous en coupe partielle des transducteurs de thérapie 20 et d'imagerie 24 des figures 5 et 6, en position d'imagerie. On a représenté sur la figure 7 le transducteur 20, en traits pleins dans sa position escamotée et en traits pointillés dans sa position de tir. Le transducteur d'imagerie est représenté en traits pleins dans sa position d'imagerie; la position rétractée n'est pas représentée car elle n'est pas dans le plan de coupe.

La figure 8 est une vue de côté de la sonde selon le deuxième mode de réalisation de l'invention, en coupe partielle et en position de tir. Comme on le voit sur la figure 8, en position de tir, le transducteur d'imagerie 24 est situé dans l'encoche 27. sur la partie inférieure du transducteur de thérapie 20.

Le fonctionnement de la sonde selon le deuxième mode de réalisation de l'invention est analogue à celui de la sonde décrite dans FR-A-2 708 207, à l'exception des points suivants.

D'une part, il n'est pas nécessaire de prévoir des moyens d'entraînement pour le transducteur de thérapie 20. Le transducteur de thérapie 20 passe de sa position de tir à la position escamotée sous l'action du transducteur d'imagerie, lorsque celui-ci passe de la position rétractée à la position d'imagerie. Plus précisément, lorsque le transducteur d'imagerie passe de la position rétractée à la position d'imagerie, il repousse le transducteur à l'encontre des moyens de rappel. Lorsque le transducteur d'imagerie est ramené en position rétractée les moyens de rappel ramènent le transducteur en position de tir. On obtient ainsi les avantages de simplicité de structure et de fonctionnement déjà décrits en référence au premier mode de réalisation de l'invention.

D'autre part, la présence de l'encoche 27 permet que la position rétractée du transducteur d'imagerie soit proche du transducteur, comme représenté sur la figure 8; ainsi par rapport à la sonde du document FR-A-2 708 207, l'invention permet une meilleure observation de l'organe à traiter à l'aide du transducteur d'imagerie, même pendant le traitement. Ceci apparaît sur la figure 8 où sont représentés schématiquement les zones insonifiées par le transducteur de thérapie et par le transducteur d'imagerie. En outre, elle limite l'amplitude angulaire du mouvement du transducteur de thérapie, ce qui permet de traiter dans un volume plus restreint et évite de déplacer l'organe à traiter. Enfin, dans le cas du traitement de la prostate, si le transducteur d'imagerie en position rétractée est trop éloigné du transducteur, comme ce peut être le cas dans la sonde de FR-A-2 708 207, il se trouve à la hauteur de l'anus, soit en face de tissus peu transparents aux ultrasons. Ceci est évité grâce à l'encoche selon l'invention.

Bien entendu, chacun de ces deux aspects de l'invention - moyens de rappel du transducteur ou encoche dans sa partie inférieure - est susceptible d'être mis en oeuvre indépendamment de l'autre. On peut ainsi prévoir une encoche ou fraisage aussi dans le mode de réalisation des figures 1 à 4, ou se passer d'encoche dans le mode de réalisation des figures 5 à 8.

La figure 9 montre une vue schématique en coupe d'une sonde de l'art antérieur, en position de tir et en position d'imagerie; la coupe est dans un plan sensiblement perpendiculaire à l'axe longitudinal de la sonde, et contenant l'organe à traiter. La. figure 9 montre la sonde selon FR-A-2 708 207, en traits pleins en position d'imagerie et en traits pointillés en position de tir. On reconnaît l'organe à traiter 30, par exemple la prostate pour un traitement par voie rectale, le transducteur de thérapie 31. mobile autour d'un arbre 32, le transducteur d'imagerie 33, et le ballonnet enveloppant la sonde. Comme expliqué dans le document précité, le ballonnet est gonflé avec un liquide tel que de l'eau dégazée pour venir au contact de la paroi de l'organe à traiter et assurer le passage des ultrasons. En position d'imagerie, le praticien repère la prostate, et dessine les contours de la zone à traiter; puis il passe en position de tir. En position de tir, le transducteur d'imagerie est rétracté dans le corps de sonde, et le transducteur de thérapie est basculé de 90° autour de l'arbre 32 pour venir face à l'organe à traiter, dans la position représentée en pointillés sur la figure 9. Comme représenté sur la figure 9, du fait du déplacement des transducteur, il est possible que l'organe à traiter n'occupe pas exactement la même position dans les positions de tir et d'imagerie de la sonde connue; ceci provoque des erreurs de repérage et on risque de traiter des zones saines, ou de ne pas traiter des zones malades. Dans le cas d'un traitement de la prostate, il existe donc des risques de brûlure de la paroi rectale.

L'invention propose une solution à ce problème nouvellement découvert.

Il est clair que ce problème ne se pose pas uniquement si la sonde comporte un transducteur de thérapie et un transducteur d'imagerie mobiles. Il se pose aussi si la sonde ne comprend qu'un transducteur de thérapie mobile; ceci peut être le cas par exemple pour assurer le balayage de la cible, même si la sonde ne présente pas de transducteur d'imagerie.

La figure 10 montre une vue schématique en coupe d'une sonde selon un troisième mode de réalisation de l'invention, en position de tir et en position d'imagerie; cette vue correspond à la vue de la figure 9, et illustre la solution de l'invention au problème nouveau exposé en référence à la figure 9. Les numéros de référence identiques à ceux de la figure 9 désignent les éléments identiques ou correspondants.

Selon l'invention, la sonde présente un anneau de garde 35, qui maintient l'organe à traiter 30 et le ballonnet ou enveloppe 34 dans la même position lorsque les transducteurs de thérapie 31 et d'imagerie 33 sont déplacés pour passer de la position d'imagerie à la position de thérapie, et inversement. L'anneau de garde 35 permet d'éviter les mouvements de l'organe à traiter, et assure que la position relative de la sonde et de l'organe à traiter reste la même, que la sonde soit en position de tir ou en position de traitement. Plus précisément l'anneau de garde limite les déformations de l'enveloppe ou ballonnet lorsque le transducteur de thérapie se déplace.

L'anneau de garde est avantageusement conformé de sorte à présenter un contour proche de celui du transducteur de thérapie: ceci assure une section minimale de la sonde dans sa position de tir, et permet une introduction facile dans une cavité. On conserve ainsi un transducteur de thérapie présentant une surface importante, comme dans FR-A-2 708 207, en évitant de devoir augmenter la taille de la sonde.

L'anneau de garde peut présenter la forme d'un anneau entourant le transducteur de thérapie, comme le suggère son nom et comme représenté à la figure 11. Dans ce cas. il peut être réalisé dans un matériau quelconque, par exemple en tôle emboutie, et présenter un profil proche de celui du transducteur de thérapie. comme cela apparaît sur les figures 1 à 4. L'anneau de garde peut aussi présenter la forme d'un disque, présentant au moins en son centre une fenêtre acoustiquement transparente, permettant de laisser passer les ondes de thérapie ou d'imagerie. On peut par exemple réaliser l'anneau de garde en métal, ou en tout matériau suffisamment rigide pour assurer un maintien de l'organe. Dans ce cas, il est possible que le ballonnet ne s'étende que sur l'arrière de l'anneau de garde, l'anneau de garde lui-même constituant la paroi de la sonde au contact de l'organe à traiter. Ceci évite encore plus que l'organe à traiter ne se déplace lors du gonflage du ballonnet. Dans un tel cas, l'enveloppe du transducteur de thérapie n'est que partiellement déformable, du même côté de l'anneau de garde que le transducteur. On peut aussi prévoir que l'enveloppe est fixée sur l'anneau de garde.

La figure 11 montre une vue schématique d'un quatrième mode de réalisation de l'invention; on reconnaît sur la figure 11 le corps de sonde 41, le transducteur de thérapie 42, le transducteur d'imagerie 43; la flèche 44 indique le déplacement du transducteur d'imagerie en translation selon un axe parallèle à l'axe longitudinal de la sonde, et la flèche 45 indique le déplacement du transducteur de thérapie en rotation autour de l'arbre 46.

Dans le mode de réalisation de la figure 11, l'anneau de garde 47 présente l'allure d'un anneau dont le contour est sensiblement celui du transducteur de thérapie 42; l'anneau de garde 47 est monté sur les moyens de support de la sonde. et le corps de sonde 41 est libre en rotation par rapport à l'anneau de garde. Cette solution présente l'avantage d'assurer une immobilité parfaite de l'organe à traiter par rapport à l'anneau de garde, même si l'on fait tourner le corps de sonde en rotation autour de son axe longitudinal pour un balayage latéral en thérapie.

La figure 12 montre une vue schématique d'un cinquième mode de réalisation de l'invention: ce mode de réalisation est sensiblement identique à celui de la figure 11, à l'exception du fait que l'anneau de garde 48 est monté sur l'extrémité frontale du corps de sonde. Cette solution présente les avantages d'une grande simplicité de mise en oeuvre. et d'assurer une section minimale à la sonde. Il est toutefois possible dans ce cas que l'organe à traiter bouge légèrement si l'on fait tourner le corps de sonde en rotation autour de son axe longitudinal pour un balayage latéral en thérapie. comme représenté sur la figure 13, en traits pleins et en traits pointillés. Ce mouvement est en tout état de cause d'un amplitude considérablement limitée par rapport aux mouvements possibles dans la solution de l'art antérieur décrite en référence à la figure 9.

Bien entendu, la solution de l'invention peut être mise ne oeuvre avec les autres modes de réalisation de l'invention, seule ou en combinaison. Ainsi, on peut prévoir un anneau de garde dans le premier mode de réalisation de l'invention, comme représenté sur les figues 1 à 4; on pourrait aussi ne pas le prévoir, du fait des mouvements limités du transducteur de thérapie dans ce mode de réalisation. Dans le deuxième mode de réalisation de l'invention, on peut avantageusement prévoir aussi un anneau de garde, même si le déplacement angulaire du transducteur de thérapie est limité.

La présence d'un anneau de garde est particulièrement avantageuse lorsque, comme expliqué en référence aux figures 1 à 8, le transducteur de thérapie est monté sur des moyens de rappel, et passe de sa position de thérapie à sa position escamotée sous l'action transducteur d'imagerie lorsque celui-ci passe de sa position rétractée à sa position d'imagerie. Lorsque l'on rétracte le transducteur d'imagerie, l'anneau de garde peut servir de butée au transducteur de thérapie, et assurer ainsi son positionnement précis. On peut alors prévoir des moyens de maintien du transducteur de thérapie sur l'anneau de garde, permettant de maintenir le transducteur de thérapie contre l'anneau de garde, ou encore des moyens de détection, permettant de détecter la position du transducteur de thérapie contre l'anneau de garde, pour autoriser les tirs.

En tout état de cause, la présence d'un anneau de garde est avantageuse dès qu'il est nécessaire d'assurer le maintien de l'organe à traiter lors des mouvements d'un transducteur. L'anneau de garde permet de limiter les déformations d'une enveloppe au moins partiellement déformable qui entoure un transducteur de thérapie mobile. On assure ainsi un bon maintien de l'organe à traiter, et une section minimale de la sonde.

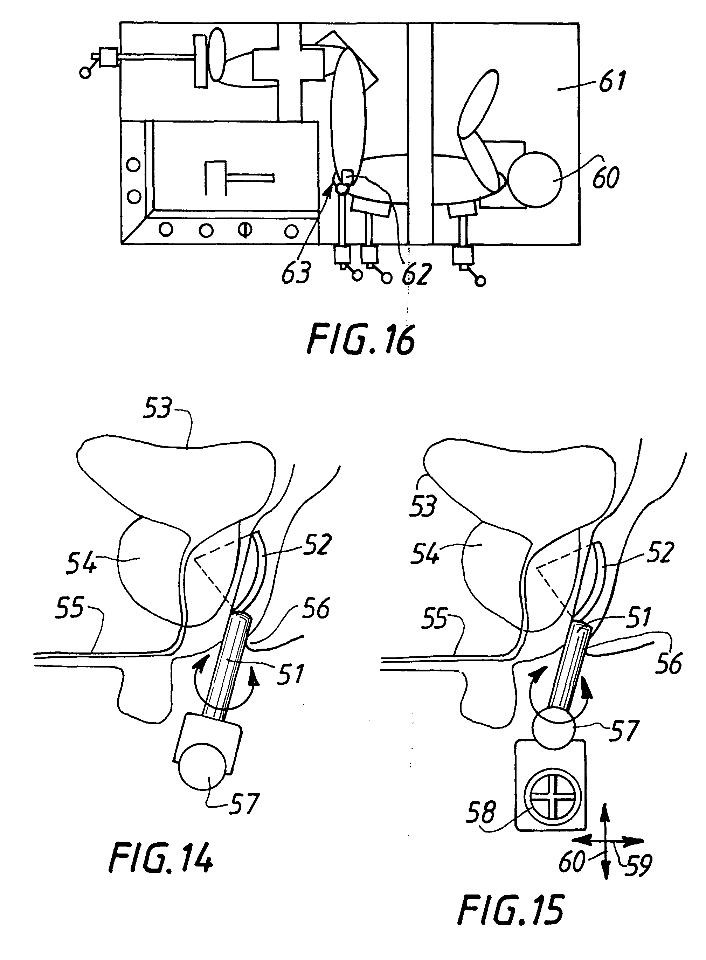

Les figures 14 et 15 montrent deux vues schématiques en coupe de sondes selon l'invention, en position pour un traitement de la prostate par voie rectale; on reconnaît sur les figures 14 et 15 la sonde 51, avec le transducteur de thérapie 52, la vessie 53, la prostate 54, l'urètre 55 et le rectum 56 du patient. Les figures 14 et 15 sont des vues de dessus, le patient étant normalement pour ce type de traitement couché en chien de fusil sur une table de traitement.

Selon l'invention, la sonde 51 est montée sur une rotule 57, ce qui permet une introduction plus facile dans le rectum: grâce à la rotule 57, on peut disposer la sonde de telle sorte que son axe longitudinal soit précisément parallèle au rectum du patient. On évite ainsi de blesser le patient et on facilite l'introduction de la sonde.

Comme on le voit sur la figure 15, la rotule 57 de la sonde 51 est avantageusement montée sur des moyens de déplacement 58 permettant de déplacer la sonde en translation suivant trois axes, dont deux sont symbolisés par les flèches 59 et 60 sur la figure 15, le troisième axe étant perpendiculaire au plan de la figure. i. e. au plan de la table de traitement.

L'invention propose donc de prévoir notamment un mouvement vertical de la sonde, i. e. un mouvement dans une direction transversale à l'axe longitudinal de la sonde et transversale à la direction de propagation principale des ondes de thérapie. Ce mouvement dans une direction perpendiculaire à l'axe des figures permet de traiter des volumes plus importants, en limitant la rotation de la sonde. Dans le cas de la prostate, ce mouvement permet de bien ajuster la position de la sonde pour traiter les lobes de l'organe, sans avoir à effectuer des déplacements importants de la sonde en rotation autour de son axe longitudinal.

Ce mouvement peut être motorisé ou non. Dans le cas ou le mouvement est motorisé, on peut programmer le déplacement motorisé transversalement à la direction de propagation principale des ondes de thérapie pour effectuer un balayage de l'organe à traiter, en évitant ou en limitant les rotation du corps de sonde autour de son axe.

Si le déplacement n'est pas motorisé, il permet néanmoins, pour des organes de taille importantes, d'effectuer un réglage manuel en milieu de traitement, de sorte à limiter l'amplitude de la rotation de la sonde.

L'invention permet ainsi de conserver tout au long du traitement un champ de traitement dont la direction de propagation est toujours sensiblement perpendiculaire à la surface de l'organe à traiter.

Dans le plan des figures 14 et 15, i.e. dans le plan contenant la direction de propagation principale des ondes de thérapie, le déplacement peut être motorisé ou non. Dans un mode de réalisation de l'invention, le déplacement est motorisé, mais la sonde est aussi susceptible d'être déplacée manuellement. Ceci présente les avantages suivants. D'une part. à l'introduction de la sonde, ceci permet au praticien de bien contrôler le positionnement de la sonde, et de mesurer la résistance à l'introduction. On évite de blesser le patient par le mouvement motorisé de la sonde, dont la puissance n'est pas nécessairement bien contrôlée et perçue par le praticien. D'autre part, le déplacement manuel de la sonde permet de constituer une référence pour limiter ensuite les déplacements motorisés de la sonde. Ainsi la position initiale déterminée manuellement correspond à la position médiane, et les moteurs ne devraient pas déplacer la sonde par rapport à cette position d'une quantité supérieure à une valeur prédéterminée.

Dans un mode de réalisation particulièrement avantageux de l'invention. le déplacement de la sonde au cours du traitement est assuré avec des déplacements différents dans les différentes directions.

En effet, on peut traiter un volume en juxtaposant des lésions élémentaires, i.e. des tirs élémentaires, en déplaçant le transducteur suivant les deux directions de balayage du volume à traiter. Ces deux directions sont les directions généralement perpendiculaires à la direction de propagation du faisceau, et correspondent à un mode de traitement dans lequel on traite le volume par plan successifs sensiblement perpendiculaires à la direction de propagation des ondes.

Ce mode de traitement résoud le problème nouveau suivant: il est possible que le transducteur de thérapie génère un faisceau asymétrique, notamment s'il présente une forme rectangulaire ou une forme telle que celle de la figure 1. Dans ce cas, à cause de l'asymétrie du faisceau ultrasonore, la juxtaposition de lésions avec un pas constant dans les 2 directions entraîne soit une superposition des lésions suivant une direction, soit une zone intermédiaire non traitée dans l'autre direction.

Le faisceau ultrasonore n'est pas nécessairement symétrique en fonction de la forme du transducteur; dans le cas d'une forme rectangulaire du transducteur de thérapie, les lésions induite au point focal sont donc de section elliptique, avec le plus petit diamètre correspondant à la plus grande largeur du transducteur.

Pour résoudre ce problème nouveau, particulièrement pour des transducteurs à tache focale allongée, tels que des transducteurs rectangulaires, l'invention propose d'adapter le pas de tir à la forme de la lésion. Par exemple pour un transducteur rectangulaire de 50 mm x 35 mm, le pas de tir optimal est de 1,6 mm dans la direction longitudinale et de 2,3 mm dans la direction latérale.

L'invention propose donc que des moyens de déplacement de la sonde déplacent la sonde avec des pas différents dans les différentes directions. Ce mode de réalisation de l'invention peut être mis en oeuvre indépendamment des autres modes de réalisation de l'invention.

La figure 16 montre une vue schématique de dessus d'un patient en position de traitement par une sonde selon l'invention. La figure 16 montre le patient 60, sur une table de traitement 61, le cas échéant avec des coussins. Le patient est maintenu immobile par des sangles ou cales. Malgré cela, il est possible que le patient, au cours du traitement, bouge. Ceci risque de déplacer la sonde par rapport au patient, et conduire à un traitement incomplet ou inapproprié.

Pour en avertir le praticien, et lui permettre de vérifier l'adéquation du traitement, l'invention propose de munir le patient d'une barrière optique, permettant de repérer les mouvements du patient. A cet effet, on fixe sur le patient, par exemple sur la hanche, un réflecteur 62, et on prévoit sur la table de traitement 61, ou sur un point fixe par rapport à la table. un émetteur récepteur 63. On peut utiliser comme réflecteur ou comme émetteur récepteur des éléments connus de l'homme du métier. La barrière optique est sur la figure 16 disposée dans un plan perpendiculaire au plan de la table de traitement, de sorte à détecter les mouvements du patient dans la direction de la table de traitement. Ces mouvements sont en effet les plus fréquents.

L'invention permet d'assurer un bon maintien du patient, et on peut alors considérer que l'organe à traiter - ici la prostate- est fixe dans l'espace. ceci permet d'utiliser une sonde montée sur un ensemble de motorisation fixe par rapport à la table de traitement. On peut ainsi commander avec précision les déplacements de la sonde par rapport à l'organe à traiter.

Il est clair que l'invention ne s'applique pas seulement pour le traitement, mais aussi avant et après le traitement, en dehors des phase de traitement proprement dite. Elle peut s'appliquer aussi simplement pour l'imagerie, et le terme "table de traitement" n'implique pas que le patient soit effectivement nécessairement en train de subir un traitement lorsque l'invention est appliquée. L'invention n'est donc pas une méthode de traitement thérapeutique du corps humain.

Dans un autre mode de réalisation avantageux, la sonde de l'invention comprend des moyens de circulation du liquide de gonflage de l'enveloppe entourant le transducteur de thérapie. Ces moyens de circulation comprennent par exemple au moins deux canaux ménagés dans le corps de sonde, et permettant de faire circuler le liquide de couplage remplissant l'enveloppe. On couple alors la sonde à une pompe de circulation et à un réservoir de liquide, ou à des moyens équivalents.

Par rapport aux solutions de l'art antérieur précité, dans lesquelles un canal unique de gonflage de l'enveloppe est prévu, l'invention permet par la circulation du liquide d'évacuer les bulles se formant éventuellement en cours de traitement, et/ou de permettre le refroidissement du ou des transducteurs et/ou de la paroi de l'organe à traiter.

En effet, des bulles peuvent se former dans le liquide, par échauffement du liquide ou encore du fait de la cavitation que peuvent engendrer les ondes émises par le transducteur. La circulation du liquide permet de limiter la formation de ces bulles, ce qui améliore l'efficacité du traitement, et limite l'échauffement de la sonde.

Avantageusement, le liquide est thermostaté: la circulation de liquide thermostaté peut constituer un moyen simple et efficace de refroidir le transducteur de thérapie ou le transducteur d'imagerie. La circulation de fluide permet aussi de refroidir ou de maintenir à une température basse la partie de l'organe à traiter au contact de la sonde. On limite ainsi les brulûres superficielles du patient; dans le cas d'un traitement de la prostate, on évite les brulûres de la paroi rectale.

Bien entendu, les modes de réalisation préférés de l'invention sont susceptibles de variantes: on peut combiner les différentes caractéristiques des différents modes de réalisation décrits ou les utiliser seules, selon le besoin.

L'application de la sonde au traitement de la prostate n'est qu'un exemple des traitements possibles. La sonde de l'invention peut aussi être employée pour les traitements mentionnés plus haut.

## Revendications

1. Sonde endocavitaire de thérapie, comprenant un transducteur de thérapie (2; 31; 42) monté mobile par rapport à un corps de sonde (1; 41) entre une position de thérapie et une position escamotée, dans une enveloppe (34) au moins partiellement déformable lorsque le transducteur se déplace, **caractérisée par** un anneau de garde (11; 35, 47) enveloppé dans l'enveloppe limitant en amplitude les déformations de l'enveloppe produites au voisinage de la position de thérapie du transducteur lorsque le transducteur se déplace par rapport au corps de sonde de la position de thérapie à la position escamotée.

2. Sonde selon la revendication 1, **caractérisée en ce que** l'anneau de garde constitue une butée sur laquelle le transducteur de thérapie vient en appui en position de thérapie.

3. Sonde selon la revendication 2, **caractérisée par** des moyens (12) de maintien du transducteur en position de thérapie (2) contre l'anneau de garde (11).

4. Sonde selon l'une des revendications 1 à 3, **caractérisée en ce que** l'anneau de garde a la forme d'un anneau entourant le transducteur de thérapie en position de thérapie.

5. Sonde selon l'une des revendications 1 à 3, **caractérisée en ce que** l'anneau de garde a la forme d'un disque recouvrant sensiblement le transducteur de thérapie en position de thérapie.

6. Sonde selon l'une des revendications 1 à 5, **caractérisée en ce que** l'anneau de garde est monté sur le corps de sonde.

7. Sonde selon l'une des revendications 1 à 5, **caractérisée en ce que** l'anneau de garde est monté sur un support du corps de sonde.

8. Sonde selon l'une des revendications 1 à 7, **caractérisée en ce qu'**elle comprend un transducteur d'imagerie (3; 24), monté mobile sur le corps de sonde (1; 21), et en ce **en ce que** le transducteur d'imagerie est mobile entre une position rétractée et une position d'imagerie dans laquelle le transducteur de thérapie est en position escamotée.

9. Sonde selon la revendication 8, **caractérisée en ce que** le transducteur de thérapie passe de sa position de thérapie à sa position escamotée sous l'action du transducteur d'imagerie lorsque celui-ci passe de sa position rétractée à sa position d'imagerie,
et **en ce que** le transducteur de thérapie passe de sa position escamotée à sa position de thérapie sous l'action de moyens de rappel (8, 9, 10) lorsque le transducteur d'imagerie passe de sa position d'imagerie à sa position rétractée.

10. Sonde selon la revendication 8 ou 9, **caractérisée en ce que** le transducteur d'imagerie (3; 24) est mobile en translation parallèlement à l'axe longitudinal de la sonde.

11. Sonde selon la revendication 8, 9 ou 10, **caractérisée en ce que** le transducteur de thérapie (2) est mobile en translation le long d'un axe sensiblement perpendiculaire à l'axe longitudinal de la sonde.

12. Sonde selon l'une des revendications 9 à 11, **caractérisée en ce que** les moyens de rappel comprennent des lames formant ressort.

13. Sonde selon la revendication 8, 9 ou 10, **caractérisée en ce que** le transducteur de thérapie (2) est mobile en rotation autour d'un axe (21; 32) sensiblement parallèle à l'axe longitudinal de la sonde.

14. Sonde selon l'une des revendications 9 à 11, **caractérisée en ce que** les moyens de rappel comprennent un ressort de torsion.

15. Sonde selon l'une des revendications 8 à 14, **caractérisée en ce que** le transducteur de thérapie (20) présente une encoche (27) pour recevoir le transducteur d'imagerie en position rétractée.

16. Sonde selon la revendication 15, **caractérisée en ce que** l'encoche (27) est ménagée de préférence par fraisage sur le côté du transducteur de thérapie le plus proche du corps de sonde.

17. Sonde selon l'une des revendications 1 à 16, **caractérisée en ce qu'**elle est montée sur une rotule (57).

18. Sonde selon l'une des revendications 1 à 17, **caractérisée en ce qu'**elle est montée sur des moyens d'entraînement en translation dans une direction sensiblement perpendiculaire à la direction principale de propagation des ondes de thérapie et sensiblement perpendiculaire à l'axe longitudinal de la sonde.

19. Sonde selon l'une des revendications 1 à 17, **caractérisée en ce qu'**elle est montée sur des moyens d'entraînement en translation dans trois directions perpendiculaires deux à deux, et **en ce que** lesdits moyens d'entraînement sont débrayables pour permettre un entraînement manuel au moins dans un plan.

20. Sonde selon l'une des revendications 1 19, **caractérisée en ce qu'**elle est montée sur des moyens de déplacement pour déplacer la sonde avec des pas différents dans les différentes directions.

21. Sonde selon l'une des revendications 1 à 20, **caractérisée en ce qu'**elle comprend des moyens de circulation d'un liquide, de préférence thermostaté, dans l'enveloppe.

22. Sonde selon la revendication 21, **caractérisée en ce que** les moyens de circulation comprennent au moins deux canaux disposés dans le corps de sonde.

## Claims

1. An endocavital therapy probe comprising a therapy transducer (2; 3; 42) movably mounted with respect to a probe body (1; 41) between a therapy position and a retracted position, in a casing (34) at least partially deformable when the transducer moves, **characterized by** a guard ring (11; 35, 47) encased in the casing, limiting the amplitude of deformation of said casing produced when the transducer moves with respect to the probe body from the therapy position to the retracted position.

2. The probe according to claim 1, **characterized in that** said guard ring constitute an end-of-travel stop against which said therapy transducer bears in a therapy position.

3. The probe according to claim 2, **characterized by** means (12) for maintaining said transducer in a therapy position (2), against said guard ring (1).

4. The probe according to any one of claims 1 to 3, **characterized in that** said guard ring has an annular shape, surrounding said therapy transducer in a therapy position.

5. The probe according to any one of claims 1 to 3, **characterized in that** said guard ring has the shape of a disc substantially covering said therapy transducer in a therapy position.

6. The probe according to any one of claims 1 to 5, **characterized in that** said guard ring is mounted on said probe body.

7. The probe according to any one of claims 1 to 5, **characterized in that** said guard ring is mounted on a support for said probe body.

8. The probe according to any one of claims 1 to 7, comprising an imaging transducer (3 ; 24 movably mounted on the probe body (1 ; 21), and said imaging transducer being movable between a retracted position and an imaging position at which said therapy transducer is in a retracted position.

9. The probe according to claim 8, **characterized in that** said therapy transducer moves from its therapy position to a retracted position due to the action of said imaging transducer when moving from its retracted position to its imaging position
and **in that** the therapy transducer moves from a retracted position to its therapy position due to the action of recall means (8, 9, 10) when said imaging transducer moves from its imaging position to its retracted position.

10. The probe according to claim 8 or 9, **characterized in that** said imaging transducer (3; 24) is movable with a linear movement, parallel to the longitudinal axis of said probe.

11. The probe according to claim 8, 9 or 10, **characterized in that** said therapy transducer (2)is movable with a linear movement along an axis substantially perpendicular to the longitudinal axis of said probe.

12. The probe according to any one of claims 9 to 11, **characterized in that** said recall means comprise thin plates forming recall springs.

13. The probe according to any one of claims 8, 9 or 10, **characterized in that** said therapy transducer (2) is movable in rotation about an axis (21; 32) substantially parallel to the longitudinal axis of said probe.

14. The probe according to any one of claims 9 to 11, **characterized in that** said recall means comprise a torsion spring.

15. The probe according to any one of claims 8 to 14, **characterized in that** said therapy transducer (20) has a recess (27) for receiving said imaging transducer in its retracted position.

16. The probe according to claim 15, **characterized in that** said recess (27) is provided by milling a side of said therapy transducer closest to said probe body.

17. The probe according to any one of claims 1 to 16, **characterized in that** said probe is mounted on a ball joint (57).

18. The probe according to any one of claims 1 to 17, **characterized in that** it is mounted on means for driving it linearly in a direction substantially perpendicular to a main direction of propagation of therapy waves, and substantially perpendicular to a longitudinal axis of the probe.

19. A probe according to any one of claims 1 to 17, **characterized in that** it is mounted on means for driving it linearly in three mutually perpendicular directions, said drive means having clutch means allowing manual drive in at least one plane.

20. The probe according to any one of claims 1 to 19, **characterized in that** it is mounted on displacing means adapted to move said probe with a pitch which is different in said different directions.

21. The probe according to any one of claims 1 to 20 comprising circulating means preferably under thermostatic control, adapted for circulating liquid inside said flexible casing.

22. The probe according to claim 21, **characterized in that** said circulation means comprise at least two channels disposed in said probe body.

## Patentansprüche

1. Endocavitäre Therapiesonde, umfassend eine Übertragungseinrichtung (2; 31; 42) für Therapiezwecke, die in Bezug auf einen Sondenkörper (1; 41) zwischen einer Therapie-Position und einer in einer Umhüllung (34) eingezogenen Position beweglich angebracht ist, wobei die Umhüllung wenigstens teilweise verformbar ist, wenn sich die Übertragungseinrichtung verschiebt, **gekennzeichnet durch** einen Schutzring (11; 35; 47), der in die Umhüllung eingeschlossen ist und die Verformungen der Umhüllung hinsichtlich der Amplitude begrenzt, die in der Nähe der Position der Übertragungseinrichtung für Therapiezwecke erzeugt werden, wenn sich die Übertragungseinrichtung in Bezug auf den Sondenkörper von der Therapie-Position in die eingezogene Position verschiebt.

2. Sonde nach Anspruch 1, **dadurch gekennzeichnet, daß** der Schutzring eine Stütze bildet, auf die die Therapie-Übertragungseinrichtung in der Therapie-Position zum Anliegen kommt.

3. Sonde nach Anspruch 2, **gekennzeichnet durch** Einrichtungen (12) zum Halten der Übertragungseinrichtung in Therapie-Position (2) gegen den Schutzring (11).

4. Sonde nach einem der Ansprüche 1 bis 3, **dadurch gekennzeichnet, daß** der Schutzring die Form eines die Übertragungseinrichtung für Therapiezwecke in der Therapie-Position umgebenden Rings hat.

5. Sonde nach einem der Ansprüche 1 bis 3, **dadurch gekennzeichnet, daß** der Schutzring die Form einer die Übertragungseinrichtung für Therapiezwecke in der Therapie-Position im wesentlichen abdeckenden Scheibe hat.

6. Sonde nach einem der Ansprüche 1 bis 5, **dadurch gekennzeichnet, daß** der Schutzring auf dem Sondenkörper angebracht ist.

7. Sonde nach einem der Ansprüche 1 bis 5, **dadurch gekennzeichnet, daß** der Schutzring auf einem Halter des Sondenkörpers angebracht ist.

8. Sonde nach einem der Ansprüche 1 bis 7, **dadurch gekennzeichnet, daß** sie eine Bild-Übertragungseinrichtung (3; 24) umfaßt, die beweglich auf dem Sondenkörper (1; 21) angebracht ist, und daß die Bild-Übertragungseinrichtung zwischen einer zurückgezogenen Position und einer Bild-Position, in der sich die Übertragungseinrichtung für Therapiezwecke in eingezogener Position befindet, beweglich ist.

9. Sonde nach Anspruch 8, **dadurch gekennzeichnet, daß** die Übertragungseinrichtung für Therapiezwecke von ihrer Therapie-Position in ihre eingezogene Position unter der Einwirkung der Bild-Übertragungseinrichtung übergeht, wenn diese von ihrer zurückgezogenen Position in ihre Bild-Position übergeht, und daß die Übertragungseinrichtung für Therapiezwecke von ihrer eingezogenen Position in ihre Therapie-Position unter Einwirkung von Rückhol-Einrichtungen (8; 9; 10) übergeht, wenn die Bild-Übertragungseinrichtung von ihrer Bild-Position in ihre zurückgezogene Position übergeht.

10. Sonde nach Anspruch 8 oder Anspruch 9, **dadurch gekennzeichnet, daß** die Bild-Übertragungseinrichtung (3; 24) bei Verschiebung parallel zur Längsachse der Sonde beweglich ist.

11. Sonde nach Anspruch 8, 9 oder 10, **dadurch gekennzeichnet, daß** die Übertragungseinrichtung (2) für Therapiezwecke bei Verschiebung entlang einer Achse beweglich ist, die im wesentlichen im rechten Winkel zur Längsachse der Sonde liegt.

12. Sonde nach einem der Ansprüche 9 bis 11, **dadurch gekennzeichnet, daß** die Rückhol-Einrichtungen Federblätter umfassen.

13. Sonde nach Anspruch 8, 9 oder 10, **dadurch gekennzeichnet, daß** die Übertragungseinrichtung (2) für Therapiezwecke bei Rotation um eine Achse (21; 32) beweglich ist, die im wesentlichen parallel zur Längsachse der Sonde ist.

14. Sonde nach einem der Ansprüche 9 bis 11, **dadurch gekennzeichnet, daß** die Rückhol-Einrichtungen eine Torsionsfeder umfassen.

15. Sonde nach einem der Ansprüche 8 bis 14, **dadurch gekennzeichnet, daß** die Übertragungseinrichtung (20) für Therapiezwecke eine Einkerbung (27) zur Aufnahme der Bild-Übertragungseinrichtung in zurückgezogener Position aufweist.

16. Sonde nach Anspruch 15, **dadurch gekennzeichnet, daß** die Einkerbung (27) vorzugsweise angebracht wird durch Einfräsen auf der Seite der Übertragungseinrichtung für Therapiezwecke, die dem Sondenkörper am nächsten liegt.

17. Sonde nach einem der Ansprüche 1 bis 16, **dadurch gekennzeichnet, daß** sie auf einem Kugelgelenk (57) angebracht ist.

18. Sonde nach einem der Ansprüche 1 bis 17, **dadurch gekennzeichnet, daß** sie auf AntriebsEinrichtungen zum Verschieben in einer Richtung abgebracht ist, die im wesentlichen senkrecht zur Haupt-Ausbreitungsrichtung der Therapie-Wellen und im wesentlichen senkrecht zur Längsachse der Sonde liegt.

19. Sonde nach einem der Ansprüche 1 bis 17, **dadurch gekennzeichnet, daß** sie auf AntriebsEinrichtungen zum Verschieben in drei jeweils paarweise senkrecht zueinander angeordneten Richtungen angebracht ist und daß die Antriebseinrichtungen entkoppelbar sind, um einen manuellen Antrieb wenigstens in einer Ebene zu ermöglichen.

20. Sonde nach einem der Ansprüche 1 bis 19, **dadurch gekennzeichnet, daß** sie auf Verschiebe-Einrichtungen zum Verschieben der Sonde in verschiedenen Schritten in den verschiedenen Richtungen angebracht ist.

21. Sonde nach einem der Ansprüche 1 bis 20, **dadurch gekennzeichnet, daß** sie Einrichtungen zum Umlaufenlassen einer Flüssigkeit, vorzugsweise einer thermostatisierten Flüssigkeit, in der Umhüllung umfaßt.

22. Sonde nach Anspruch 21, **dadurch gekennzeichnet, daß** die Einrichtungen zum Umlaufenlassen wenigstens zwei Kanäle umfassen, die in dem Sondenkörper angeordnet sind.
